# FASCICULE DE BREVET EUROPEEN

(11) **EP 2 082 744 B1**
(45) Date de publication et mention de la délivrance du brevet: **21.09.2011**
(21) Numéro de dépôt: 09151111.3
(22) Date de dépôt: 22.01.2009
(51) Int. Cl.: A61K 33/00, A61K 33/30, A61P 17/12

(54) **Composition pharmaceutique comprenant de l'acide nitrique et un sel de zinc et son dispositif médical pour le traitement de lésions cutanées**
Pharmazeutische Zusammensetzung enthaltend Salpetersäure und einen Zinksalz und entsprechende medizinische Vorrichtung für die Behandlung von Hautläsionen
Pharmaceutical composition comprising nitric acid and a zinc salt and medial device for treating skin lesions

(30) Priorité: 25.01.2008 EP 08100943
(43) Date de publication de la demande: 29.07.2009
(73) Titulaire: Auriga International, 1410 Waterloo (BE)
(72) Inventeur: Marchal, Alfred, 1410 Waterloo (BE)
(74) Mandataire: Luys, Marie-José A.H.

(56) Documents cités:
- EP-A- 0 026 532
- WO-A-95/03734
- GB-A- 2 371 491
- VALEANT PHARMACEUTICALS GERMANY GMBH: "Solco-Derman" ROTE LISTE - FACHINFORMATION, [Online] février 2006 (2006-02), pages 1-2, XP002485473 Extrait de l'Internet: URL:http://www.fachinfo.de/viewFI?FINR=004 811&RL=Solco-Derman%26reg%3B> [extrait le 2008-06-24]
- SCHWEIZERISCHES HEILMITTEL INSTITUT: SWISS MEDIC JOURNAL, [Online] juin 2002 (2002-06), pages 429-430, XP002485474 Bern, Switzerland Extrait de l'Internet: URL:http://www.swissmedic.ch/files/pdf/06_ 2002.pdf> [extrait le 2008-06-24]
- M. STREIT, L.R. BRAATHEN: "Therapie von Warzen" SCHWEIZ MED FORUM, [Online] no. 34, 22 août 2001 (2001-08-22), pages 839-847, XP002485475 Extrait de l'Internet: URL:http://www.medicalforum.ch/pdf/pdf_d/2 001/2001-34/2001-34-144.PDF> [extrait le 2008-06-24]
- HEATON C L: "The revival of nitric acid for the treatment of anogenital warts" CLINICAL PHARMACOLOGY & THERAPEUTICS, MOSBY-YEAR BOOK, ST LOUIS, MO, US, vol. 54, no. 1, 1 juillet 1993 (1993-07-01), pages 107-111, XP002111731 ISSN: 0009-9236

## Description

La présente invention se rapporte à une composition pharmaceutique pour le traitement de maladies de la peau comprenant une quantité thérapeutique en solution aqueuse d'acide nitrique de 5 à 15 mmol/ml de composition pharmaceutique.

Une telle composition est par exemple connue du document « The revival of nitric acid for the treatment of anogenital warts, C.L. Heaton et coll., Clinical Pharmacology & Therapeutics - July 1993 - 107, 111 » qui divulgue les avantages de l'utilisation d'acide nitrique dans de telles lésions.

la composition suivant l'invention comprend également un sel de cuivre à une concentration de 0,01 à 0,015 mg d'ions Cu²⁺/ml de composition pharmaceutique.

On connait du document EP 026 532 une solution aqueuse d'acide nitrique 6 à 10 M, qui contient un nitrite métallique ou un acide nitreux. La solution selon EP 026 532 permet d'obtenir une réaction chimique aussi efficace que celle de l'acide nitrique concentré, mais sans déployer l'effet caustique puissant, en particulier sur les tissus de ce dernier.

Des produits tels que divulgués dans EP 026 532 sont fabriqués par addition d'acide organique oxydable à l'acide nitrique. L'oxydation des acides organiques favorise la réduction de l'acide nitrique en favorisant la formation de produits de réduction de nitrate tels que des gaz nitreux et l'acide nitreux et des produits de condensation tels que des dérivés O-nitryle et O-nitrosyle.

Malheureusement, la solution produite selon l'enseignement du document EP 026 532 n'est pas stable au cours du temps et, en outre, son efficacité diminue lorsque la concentration en dérivé nitreux diminue.

D'après le document EP 630 650 qui décrit une amélioration du produit EP 026 532 par la même demanderesse, on apprend que l'efficacité du produit selon EP 026 532 se perd lorsque la teneur en produits de réaction mesurables en tant que nitrite diminue trop fortement. On apprend en outre que les produits obtenus selon EP 026 532 requièrent de ne pas fermer hermétiquement les emballages contenant les produits, mais de les conserver dans un récipient à fermeture lâche.

Dès lors, EP 026 532 utilise divers acides carboxyliques organiques qui s'oxydent à des vitesses différentes pour compenser une diminution de la teneur en nitrite.

D'après EP 630 650, la réaction des acides carboxyliques oxydables s'effectue trop lentement, à la température ordinaire, pour suffire à remplacer les nitrites et pour pallier le fait que la concentration en nitrite peut subir des variations considérables suivant la température et la durée de stockage, il est prévu de préparer une solution d'acide nitrique aqueux 1 à 5,5 M et d'un alcanol primaire.

D'après EP 630 650, on obtient alors une solution présentant une efficacité aussi bonne et pleinement reproductible, sans encourir les désavantages des produits déjà connus, grâce à cette solution qui permet encore de réduire légèrement la concentration en acide nitrique. Puisque la stabilité est améliorée, on réduit les risques dus aux produits devenus inefficaces qui présentent un danger accru d'effets secondaires et peuvent notamment conduire à des ulcérations de la peau saine.

EP 026 532 enseigne également l'utilisation de divers sels métalliques tels que ceux du cuivre, de l'argent, du cadmium, du zinc, de l'aluminium, du calcium, du strontium, du magnésium, du fer, de l'antimoine, du bismuth, du sélénium, du manganèse, du Zirconium, du cobalt, de l'or, du titane et du zinc et préfère les nitrates correspondants.

Parmi ces ions métalliques, les ions cuivre, argent, cadmium et zinc sont préférés sans aucune distinction entre ces derniers et sans associer aucun effet technique avantageux à leur présence.

Il est un but de l'invention de procurer une nouvelle composition pharmaceutique présentant une efficacité améliorée par rapport à celles existantes dans le traitement des maladies de la peau et des lésions cutanées et qui est également plus stable au cours du temps, sans toutefois ajouter plusieurs substances actives qui pourraient être considérées comme problématiques dans la pharmacologie moderne.

A cette fin, il est prévu suivant l'invention une composition pharmaceutique telle que mentionnée au début comprenant en outre une quantité thérapeutique en solution aqueuse
- d'acide lactique en une quantité allant de 1 à 50 mg/ml de composition pharmaceutique,
- d'acide oxalique en une quantité allant de 10 à 70 mglml de composition pharmaceutique,
- d'acide acétique en une quantité allant de 1 à 50 mg/ml de composition pharmaceutique, et
- un sel de cuivre à une concentration de 0,01 à 0,015 mg ions Cu²⁺/ml de composition pharmaceutique, caractérisée en ce qu'elle comprend en outre un sel de zinc à une concentration de 0,01 à 0,015 mg ions Zn²⁺/ml de composition pharmaceutique.

Outre son effet antiseptique et antibactérien avantageux, le cuivre conjointement avec l'acide nitrique et les nitrites dénature les protéines de la peau permet une momification des lésions ou maladies de la peau (nécrose de la zone comportant la lésion).

Le zinc a une action sur l'immunité cellulaire et il favorise donc la lutte contre les infections, en particulier dans les cellules du corps humain et donc également celles de la peau.

Ajouter du zinc à une composition pour le traitement de lésions cutanées et maladies de la peau est particulièrement avantageux de par son efficacité à augmenter la réponse immunitaire cellulaire. En outre, le zinc possède également un rôle clé dans les membranes cellulaires et joue un rôle structurel important, renforçant l'effet médiateur de la réponse immunitaire cellulaire.

Dès lors, la composition pharmaceutique selon l'invention permet de favoriser la réponse cellulaire vis-à-vis d'une infection potentielle qui pourrait accompagner la lésion cutanée, et elle renforce la structure membranaire des cellules de la peau. En outre, le zinc favorise la cicatrisation de la peau ayant subi ladite lésion, ce qui rend son rôle complètement synergique avec celui de l'acide nitrique de la composition pharmaceutique selon l'invention.

Dans de telles maladies de la peau ou lésions cutanées, les cellules de la peau prolifèrent de façon non contrôlée dans certains cas tandis que dans d'autres, ce sont des cellules mortes qui s'accumulent à la surface de la peau.

Il a donc été trouvé de manière surprenante que conjointement à l'acide nitrique qui détruit les cellules « anormales » de la lésion ou les cellules malades, le zinc avait un effet bénéfique sur la lésion.

Le résultat est particulièrement surprenant car le zinc est essentiel à la croissance cellulaire et à la multiplication des cellules. Dès lors, il était plutôt inimaginable de l'utiliser lorsqu'il faut cesser la prolifération non contrôlée ou lorsqu'il faut détruire des cellules accumulées en tumeur ou en kératoses puisqu'il sert plutôt conventionnellement à leur développement.

Il a été montré par des tests que le zinc n'avait, contrairement à ce que l'on aurait pu penser, aucun effet antagoniste à celui de l'acide nitrique et qu'en outre, la cicatrisation après destruction des cellules « anormales » était plus rapide, plus efficace et que les nouvelles cellules de peau, généralement reconnaissables, présentaient un aspect « nouveau et rouge » réduit.

Plus particulièrement, comme mentionné ci-dessus, la concentration en ions zinc et cuivre dans la composition selon l'invention est semblable afin d'éviter des compétitions entre le zinc et le cuivre (côte à côte dans le tableau périodique des éléments de Mendeliev) au sein du processus de cicatrisation. Le cuivre et le zinc sont deux éléments catalyseurs de la dégradation de l'acide nitrique au sein des tissus, mais le cuivre contribue à obtenir une nécrose par dénaturation des protéines.

Comme on l'a mentionné ci-dessus, le cuivre, conjointement avec l'acide nitrique et les nitrites dénature les protéines de la peau permet une momification des lésions ou maladies de la peau (nécrose de la zone comportant la lésion). C'est donc la présence de Zn, de Cu et NO₂ (provenant de la réduction de HNO³) qui intervient lors de l'application dans la peau en catalysant la destruction de la lésion cutanée (par exemple la verrue) par HNO₃. Ceci s'ajoute aux autres propriétés du Zn et Cu déjà mentionnées.

Le zinc, ayant des propriétés chimiques proches de celles du cuivre (masses atomiques et potentiels électropositifs semblables, etc...), a un rôle physiologique très proche de celui du cuivre, notamment vis-à-vis des interactions avec les protéines. Il est donc avantageux que ces deux éléments sous forme ionisée aient une concentration relative dans la composition selon l'invention proche l'une de l'autre car ils assurent un équilibre. Si la concentration en zinc était supérieure à celle du cuivre, le zinc pourrait prendre le rôle du cuivre dans la nécrose des tissus et le cuivre resterait en surface ce qui causerait indéniablement une hyperpigmentation des cellules de l'épiderme post-guérison. En effet, le cuivre, bien qu'ayant une action antiseptique et antibactérienne est également un catalyseur de la fabrication de mélanine, ce qui provoque l'apparition des taches brunes.

De plus, la composition selon l'invention est obtenue à partir de zinc métallique (et donc présentant un état d'oxydation nul) particulièrement approprié pour son effet de réducteur puissant qui permet de maintenir la concentration en nitrite stable au cours du temps dans la composition selon l'invention. Dès lors, la composition selon l'invention a permis d'atteindre une grande stabilité de la composition, un effet cicatrisant et antiseptique optimal tout en ayant un effet de nécrose optimal et des effets secondaires très réduits.

La composition pharmaceutique selon l'invention comprend des nitrates qui sont impliqués dans la cascade des produits de réduction du nitrate qui résulte de l'interaction de l'acide nitrique concentré en tant qu'oxydant avec des acides organiques en tant que réducteurs.

Avec l'acide nitrique, les acides organiques forment donc des produits de réduction qui permettent de contribuer à la présence de la concentration minimale en nitrite dans la composition et donc permettent, même en cas d'instabilité, de produire la concentration thérapeutique. En effet, les nitrites constituant les produits de la réduction des nitrates sont impliqués dans deux mécanismes de destructions qui sont la digestion ou l'érosion du tissu par hydrolyse acide des liens peptidiques et la dévitalisation des tissus par une réaction covalente avec les protéines du tissu en laissant l'architecture de la lésion largement conservée.

Un tel mélange d'acides organiques ajoute à la composition des composés qui sont oxydables à différentes vitesses. L'acide acétique est relativement oxydé alors que l'acide oxalique l'est très lentement. Ce mélange permet de maintenir une moyenne stable en composés nitreux, conjointement au zinc réducteur, qui a un rôle dans la dévitalisation rapide des lésions traitées topiquement (C.L. Heaton et coll.), puisque la concentration en nitrite est corrélée avec le jaunissement des tissus qui est un indicateur d'efficacité.

En outre, la présence de zinc dans la composition pharmaceutique réduit la précipitation des acides organiques lorsqu'ils sont présents, ce qui rend la présence du zinc encore plus avantageuse.

Dans une forme de réalisation préférentielle, le sel de cuivre est un nitrate de cuivre et de préférence un nitrate de cuivre(II) trihydraté.

Dans une variante selon l'invention, la composition selon l'invention comprend en outre du nitrate de zinc afin d'augmenter encore la teneur en nitrate de la composition et dès lors son efficacité à soigner les lésions cutanées.

D'autres formes de réalisation de la composition pharmaceutique selon l'invention sont mentionnées dans les revendications annexées.

L'invention se rapporte également à un procédé de fabrication d'une composition pharmaceutique pour le traitement de maladies de la peau comprenant une quantité thérapeutique en solution aqueuse d'acide nitrique de 5 à 15 mmol/ml de composition pharmaceutique comprenant:
- une addition d'une quantité de zinc métallique à une concentration de 0,01 à 0,015mg ions Zn²⁺/ml à une quantité prédéterminée d'acide nitrique concentré,
- une agitation de ladite quantité prédéterminée d'acide nitrique contenant le zinc jusqu'à dissolution de celui-ci,
- un dégagement d'hydrogène gazeux avec une réduction d'acide nitrique en acide nitreux et formation d'une première solution
- une addition d'une quantité de cuivre, en particulier sous forme d'un nitrate de cuivre, à une concentration de 0,01 à 0,015 mg ions Cu²⁺/ml,
- une addition d'au moins un autre acide carboxylique, de préférence choisi parmi de l'acide acétique, de l'acide oxalique et de l'acide lactique et une réduction dudit au moins un acide carboxylique avec formation de nitrites, et
- une dilution avec de l'eau.
   dans laquelle, ladite addition d'une quantité de cuivre est réalisée
   a) dans ladite première solution, ou
   b) dans une autre quantité prédéterminée d'acide nitrique concentré, avec formation d'une deuxième solution suivie d'un mélange de la première solution à la deuxième solution, avant ladite dilution avec de l'eau.

Comme on l'a déjà mentionné ci-avant, par le fait que la composition selon l'invention est obtenue à partir de zinc métallique (et donc présentant un état d'oxydation nul) particulièrement approprié pour son effet de réducteur puissant, la concentration en nitrite est maintenue de manière stable au cours du temps de stockage de la composition selon l'invention et cela sans ajouter de produit pharmaceutique ou de substances actives nécessitant des procédures de validations longues, couteuses et laborieuses.

Le procédé selon l'invention prévoit uniquement l'addition de zinc qui permet en outre d'atteindre une synergie avec le cuivre dans la dénaturation des protéines et la nécrose des tissus malades ainsi qu'améliorer les effets de cicatrisation.

Dans une forme de réalisation avantageuse, ladite addition de cuivre est réalisée dans ladite première solution.

Dans une variante préférentielle, ladite addition de cuivre est réalisée dans une autre quantité prédéterminée d'acide nitrique concentré avec formation d'une deuxième solution et est suivie d'un mélange de la première solution à la deuxième solution avant dilution avec de l'eau.

Dès lors les conditions réductrices qui contribuent à la concentration stable en nitrite sont maintenues au cours du temps par l'oxydation différentielle et progressive d'un ou de plusieurs de ces acides carboxyliques ajoutés selon l'invention.

D'autres formes de réalisation du procédé selon l'invention sont mentionnées dans les revendications annexées.

L'invention a également pour objet un dispositif médical comprenant au moins un applicateur et une ampoule comprenant la composition pharmaceutique selon l'invention.

L'acide nitrique contenu dans la composition pharmaceutique a un effet destructeur sur les cellules qui est avantageux sur les cellules malades mais qui serait préjudiciable s'il était appliqué sur les cellules saines entourant la lésion ou les cellules malades. De même, la présence d'ions cuivre pourrait provoquer sur les cellules saines une hyperpigmentation non désirable si l'application du produit n'est pas ciblée.

Il est donc particulièrement avantageux de commercialiser un ensemble comprenant ledit applicateur et une ampoule, de préférence en verre et scellée, qui contient ladite composition afin de permettre une application précise de la composition. En outre, il n'est pas approprié d'exposer la composition selon l'invention à l'air libre oxydant.

L'acide nitrique étant un acide corrosif, le contenir dans du verre est avantageux. En outre, une telle ampoule est aisément scellée à la flamme, l'isolant ainsi de l'atmosphère oxydante qui pourrait avoir un effet néfaste sur la cascade susdite de produit de réduction du nitrate.

Dans le cas présent, la composition selon l'invention est complètement isolée du milieu environnant et l'ampoule du dispositif selon l'invention ne permet pas le passage de gaz produit maintenant ainsi les conditions réductrices.

Dans une forme de réalisation avantageuse, ledit applicateur est une pipette de verre agencée pour prélever ladite composition pharmaceutique de l'ampoule en vue de son application sur une lésion cutanée. Cet applicateur est utile dans le cas de lésions de grande taille ou lorsque l'ampoule est un récipient contenant une quantité de composition pharmaceutique convenant pour plusieurs applications, la pipette en verre est aisément autoclavable et peut être utilisées à plusieurs reprises.

Dans une variante, ledit applicateur comprend un réservoir constitué de ladite ampoule et une extrémité d'application formée d'un capillaire ayant une extrémité choisie parmi une extrémité biseautée, une partie tronquée, un pinceau, un embout à badigeonner, un tampon applicateur, une aiguille creuse et analogues. En fonction de l'application souhaitée (lésion de petite taille ou de plus grande taille), le praticien pourra soit choisir parmi plusieurs embouts d'application ou prescrire l'embout adéquat à son patient présentant une lésion particulière.

Dans une forme de réalisation alternative, ledit applicateur comprend un réservoir agencé pour recevoir le contenu de ladite ampoule et une extrémité d'application formée d'un capillaire ayant une extrémité choisie parmi une extrémité biseautée, une partie tronquée, un pinceau, un embout à badigeonner, une aiguille creuse et analogues.

Dans cette forme de réalisation, l'utilisation du dispositif médical est particulièrement simplifié et sûr. En effet, l'ampoule est fixée audit applicateur à la façon d'une cartouche sur un stylobille, ce qui rend l'ensemble sûr. Les risques de renversement, débordement et fuites sont réduits entraînant des risques de brûlure réduits pour les mains du médecin.

Par exemple, dans le cas d'un embout applicateur, il peut être prévu qu'une simple pression sur le tampon applicateur amène la quantité adéquate de composition selon l'invention dans le capillaire et permette l'application de la solution sur la zone à traiter. Le tampon en bout de capillaire empêche alors la solution de déborder en dehors de la zone à traiter et évite tout risque de blesser le patient traité, en particulier si celui-ci bouge pendant l'application de la solution. Par exemple, dans le cas d'enfants, il est fréquent que ces derniers aient peur et bougent de manière brusque, ce qui peut avoir pour résultat qu'ils soient blessés par l'embout en verre du capillaire. Si celui-ci est muni d'un tampon applicateur imbibé de la solution selon l'invention, le risque de blessure est largement réduit.

D'autres formes de réalisation du dispositif selon l'invention sont mentionnées dans les revendications annexées.

L'invention se rapporte également à une utilisation de la composition pharmaceutique pour la fabrication d'un médicament pour le traitement de maladies de la peau ou de lésions cutanées telles que des dermatoses, des kératoses, des verrues, des condylomes, de l'eczéma, des hyperkératoses, de l'acné, du psoriasis, des lésions provenant d'infections fongiques, bactériennes ou virales et analogues.

D'autres caractéristiques, détails et avantages de l'invention ressortiront de la description donnée ci-après d'un exemple de réalisation non limitatif.

### Exemple 1 - fabrication de la composition selon l'invention

On a placé 2,75 g d'acide oxalique dihydraté (2,75 % m/V - Prolabo, PA), 5 mg de nitrate de cuivre (II) trihydraté (Merck, PA - 0,0050 % mV) dans un ballon à fermeture hermétique.

On a ensuite ajouté 35,98 g d'acide, soit 25,7 ml d'acide nitrique à 65 % (densité 1,4), 1,72 ml d'acide acétique glacial (Fisher Scientific, PA) et de l'acide lactique DL à raison de 167 µl (ALFA AESAR, ACS, 85 à 90 %).

On a ensuite dissous dans un autre ballon 1,36 mg de Zn métallique (Merck très pur) dans 20 ml d'acide nitrique à 65 % (densité 1,4) et on a maintenu l'agitation jusqu'à la dissolution complète du zinc.

Le contenu du premier ballon a été ajouté à celui du deuxième et le volume a été porté à 100 ml avec de l'eau et le ballon a été obturé hermétiquement et on a mélangé pendant 15 minutes. La solution a ensuite été refroidie jusqu'à la température ambiante.

Des ampoules autocassables de 200 µl ont ensuite été remplies et scellées à la flamme.

Les ampoules sont à utiliser de la façon suivante.

L'embout doit être cassé et l'ampoule peut alors être fixée à l'applicateur.

La lésion cutanée sera préalablement nettoyée avec un désinfectant conventionnel et la composition pharmaceutique selon l'invention sera appliquée directement avec l'applicateur sur la lésion.

L'extrémité biseautée permet d'appliquer de petites quantités alors que l'extrémité tronquée ou le pinceau permet de traiter des plus grandes lésions. Pour traiter des surfaces de 2 à 3 cm², il est préférable d'utiliser la pipette en verre.

On fera ensuite pénétrer la solution par une légère pression de l'applicateur sur la lésion qui permet de faire pénétrer la composition selon l'invention uniquement dans l'épiderme et le derme et polymérise dans les cellules basales de la peau, rendant la pénétration de la composition plus en avant impossible. Dès lors, l'application répétée au même endroit n'atteint, comme on le recherche que les couches supérieures de la peau, sans endommager les cellules basale du tissu.

### Exemple 2 - Evaluation de l'efficacité et la tolérance de la composition selon l'invention comme traitement anti-verreux

On a sélectionné 23 patients âgés de 18 à 63 ans avec une moyenne d'âge de 35,9 ans pour une étude monocentrique. Les patients présentaient chacun une à plusieurs verrues. Cette étude a duré 35 jours. La composition selon l'invention (contenant 6,6 mmol/ml d'HNO₃, 42 mg/ml d'acide acétique, 35,7 mg/ml d'acide oxalique soit 0,397 mmol/ml, 4,5 mg/ml d'acide lactique, ions Cu²⁺ et 0,0134 mg/ml d'ions Zn²⁺) a été appliquée après nettoyage à l'alcool et scarification par le médecin sur la ou les verrues à raison de 1 à 3 applications à intervalle d'une semaine. Le nombre d'applications de la composition selon l'invention a été laissé à l'appréciation du médecin chargé de la présente étude clinique.

### Evaluation de l'efficacité

L'efficacité de la composition selon l'invention a été évaluée selon une estimation clinique des patients en fin d'étude (après 35 jours). Le médecin chargé de la présente étude a examiné visuellement la régression de la ou des verrues sur une échelle de 0 à 2 (0 représentant le cas où aucun effet de la composition selon l'invention n'est observé, 1 où la verrue disparaissait partiellement et 2 lorsque la disparition de la verrue était totale.

### Les résultats sont illustrés au tableau 1

**Tableau 1**

| Nombre de | Guérison totale | Guérison | Aucun effet |
|---|---|---|---|
| patients | | partielle | |
| 23 | 12 | 8 | 3 |

Comme on peut le constater, seul 3 patients n'ont pas du tout répondu au traitement après trois applications. On a pu observer une guérison totale dans 52 % des cas (12 patients) et que 8 patients (34%) ont répondu au traitement partiellement. Selon les médecins chargés de l'étude, si le traitement par la composition selon l'invention avait comporté des applications supplémentaires, le nombre de guérisons totales aurait encore augmenté.

Dans le cas des patients ayant montré une guérison totale, on a également mesuré après combien d'application la guérison totale était observée. Les résultats sont illustrés au tableau 2.

**Tableau 2**

| Nombre de | 1 application | 2 applications | 3 applications |
|---|---|---|---|
| patients | | espacées d'une | espacées d'une |
| | | semaine | semaine |
| 12 | 2 | 6 | 4 |

Comme on peut le constater, dans 50% des cas, 2 applications espacées d'une semaine ont suffit pour obtenir une guérison totale des verrues.

De même, l'influence de la localisation des verrues par rapport au résultat de guérison et le nombre d'application nécessaire à cette guérison a été étudiée. Aucune influence de la localisation des verrues, ni sur l'efficacité de la solution selon l'invention, ni sur le nombre d'application nécessaire à la guérison totale des verrues.

### Evaluation de la tolérance

Dans l'ensemble, la composition selon l'invention a été très bien tolérée. Certains patients ont ressenti soit des picotements, soit une brûlure, mais les médecins chargés de la présente étude ont conclu à une très bonne tolérance de la composition selon l'invention par les patients.

Dans un autre exemple, il est également prévu suivant l'invention que la composition comprenne également de l'argent sous forme ionisée pour son effet antiseptique majeur. Dans ce cas l'argent contribue au bon déroulement de la cicatrisation en préservant le tissu en cours de cicatrisation de toute infection.

De manière similaire, les acides carboxyliques organiques oxydants préférentiels utilisés dans la composition selon l'invention sont l'acide acétique, l'acide lactique, et l'acide oxalique, mais il va de soi que d'autres, tels que l'acide glycolique, l'acide pyruvique, l'acide glycoxylique ou encore l'acide malique et analogue peuvent également être utilisés.

## Revendications

1. Composition pharmaceutique pour le traitement de maladies de la peau comprenant une quantité thérapeutique en solution aqueuse
- d'acide nitrique de 5 à 15 mmol/ml de composition pharmaceutique,
- d'acide lactique en une quantité allant de 1 à 50 mg/ml de composition pharmaceutique,
- d'acide oxalique en une quantité allant de 10 à 70 mg/ml de composition pharmaceutique,
- d'acide acétique en une quantité allant de 1 à 50 mg/ml de composition pharmaceutique, et
- un sel de cuivre à une concentration de 0,01 à 0,015 mg ions Cu²⁺/ml de composition pharmaceutique, **caractérisée en ce qu'**elle comprend en outre un sel de zinc à une concentration de 0,01 à 0,015 mg ions Zn²⁺/ml de composition pharmaceutique.

2. Composition pharmaceutique selon la revendication 1, dans laquelle ledit sel de cuivre est un nitrate de cuivre et de préférence un nitrate de cuivre(II) trihydraté.

3. Composition pharmaceutique selon la revendication 1 ou la revendication 2, comprenant en outre du nitrate de zinc.

4. Procédé de fabrication d'une composition pharmaceutique pour le traitement de maladies de la peau comprenant une quantité thérapeutique en solution aqueuse d'acide nitrique de 5 à 15 mmol/ml de composition pharmaceutique comprenant:
- une addition d'une quantité de zinc métallique à une concentration de 0,01 à 0,015 mg ions Zn²⁺/ml à une quantité prédéterminée d'acide nitrique concentré,
- une agitation de ladite quantité prédéterminée d'acide nitrique contenant le zinc jusqu'à dissolution de celui-ci,
- un dégagement d'hydrogène gazeux avec une réduction d'acide nitrique en acide nitreux et formation d'une première solution
- une addition d'une quantité de cuivre, en particulier sous forme d'un nitrate de cuivre à une concentration de 0,01 à 0,015 mg ions Cu²⁺/ml,
- une addition d'au moins un autre acide carboxylique, de préférence choisi parmi de l'acide acétique, de l'acide oxalique et de l'acide lactique et une réduction dudit au moins un acide carboxylique avec formation de nitrites, et
- une dilution avec de l'eau.
dans laquelle, ladite addition d'une quantité de cuivre est réalisée
a) dans ladite première solution, ou
b) dans une autre quantité prédéterminée d'acide nitrique concentré, avec formation d'une deuxième solution suivie d'un mélange de la première solution à la deuxième solution, avant ladite dilution avec de l'eau.

5. Dispositif médical comprenant un applicateur et une ampoule, ladite ampoule comprenant une composition pharmaceutique selon l'une quelconque des revendications 1 à 3.

6. Dispositif médical selon la revendication 5, dans lequel ledit applicateur est une pipette de verre agencée pour prélever ladite composition pharmaceutique de l'ampoule en vue de son application sur une lésion cutanée.

7. Dispositif médical selon la revendication 5, dans lequel ledit applicateur comprend un réservoir constitué de ladite ampoule et une extrémité d'application formée d'un capillaire ayant une extrémité choisie parmi une extrémité biseautée, une partie tronquée, un pinceau, un embout à badigeonner, un tampon applicateur, une aiguille creuse.

8. Dispositif médical selon la revendication 5, dans lequel ledit applicateur comprend un réservoir agencé pour recevoir le contenu de ladite ampoule et une extrémité d'application formée d'un capillaire ayant une extrémité choisie parmi une extrémité biseautée, une partie tronquée, un pinceau, un embout à badigeonner, une aiguille creuse.

9. Composition pharmaceutique selon l'une quelconque des revendications 1 à 3 pour utilisation pour le traitement de maladies de la peau ou de lésions cutanées telles que des dermatoses, des kératoses, des verrues, des condylomes, de l'eczéma, des hyperkératoses, de l'acné, du psoriasis, des lésions provenant d'infections fongiques, bactériennes ou virales.

## Claims

1. Pharmaceutical composition for treating skin diseases comprising a therapeutic quantity, in aqueous solution, of:
- nitric acid at 5 to 15 mmol/ml of pharmaceutical composition,
- lactic acid in a quantity ranging from 1 to 50 mg/ml of pharmaceutical composition,
- oxalic acid in a quantity ranging from 10 to 70 mg/ml of pharmaceutical composition,
- acetic acid in a quantity ranging from 1 to 50 mg/ml of pharmaceutical composition, and
- a copper salt in a concentration of 0.01 to 0.015 mg of Cu^{2+/}ml ions of pharmaceutical composition, **characterized in that** it further comprises a zinc salt at a concentration of 0.01 to 0.015 mg of Zn^{2+/}ml ions of pharmaceutical composition.

2. The pharmaceutical composition according to claim 1 wherein the said copper salt is a copper nitrate, and preferably a copper (II) nitrate trihydrate.

3. The pharmaceutical composition according to claim 1 or claim 2, further comprising zinc nitrate.

4. A method for producing a pharmaceutical composition for treating skin diseases comprising a therapeutic quantity in aqueous solution of nitric acid at 5 to 15 mmol.ml of pharmaceutical composition, comprising:
- adding a quantity of metallic zinc at a concentration of 0.01 to 0.015 mg Zn^{2+/}ml ions to a predetermined quantity of concentrated nitric acid,
- stirring the said predetermined quantity of nitric acid containing the zinc until dissolution thereof,
- release of hydrogen gas with reduction of nitric acid to nitrous acid and formation of a first solution;
- adding a quantity of copper, in particular in the form of a copper nitrate at a concentration of 0.01 to 0.015 mg Cu^{2+/}ml ions,
- adding at least one other carboxylic acid, preferably chosen from among acetic acid, oxalic acid and lactic acid and reducing the said at least one carboxylic acid with formation of nitrites, and
- diluting with water
wherein the said addition of a quantity of copper is conducted:
a) in the said first solution, or
b) in another predetermined quantity of concentrated nitric acid, with formation of a second solution followed by mixing the first solution with the second solution, before the said dilution with water.

5. A medical device comprising an applicator and an ampoule, the said ampoule comprising a pharmaceutical composition according to any of claims 1 to 3.

6. The medical device according to claim 5, wherein the said applicator is a glass pipette arranged to pipette said pharmaceutical composition from the ampoule with a view to application thereof to a skin lesion.

7. The medical device according to claim 5, wherein the said applicator comprises a reservoir formed of said ampoule and an applicator tip formed of a capillary having a tip chosen from among a bevelled tip, a flattened part, a brush, a coating nozzle, an applicator swab, a hollow needle.

8. The medical device according to claim 5, wherein the said applicator comprises a reservoir arranged to receive the content of the said ampoule and an application tip formed of a capillary having a tip chosen from among a bevelled tip, a flattened part, a brush, a coating nozzle, a hollow needle.

9. The pharmaceutical composition according to any of claims 1 to 3 for use in the treatment of skin diseases or skin lesions such as dermatitis, keratosis, warts, condyloma, eczema, hyperkeratosis, acne, psoriasis, lesions due to fungal, bacterial or viral infections.

## Patentansprüche

1. Pharmazeutische Zusammensetzung zur Behandlung von Hautkrankheiten, enthaltend, in wässriger Lösung, eine therapeutische Menge an
- Salpetersäure mit 5 bis 15 mmol/ml der pharmazeutischen Zusammensetzung,
- Milchsäure in einer Menge im Bereich von 1 bis 50 mg/ml der pharmazeutischen Zusammensetzung,
- Oxalsäure in einer Menge im Bereich von 10 bis 70 mg/ml der pharmazeutischen Zusammensetzung,
- Essigsäure in einer Menge im Bereich von 1 bis 50 mg/ml der pharmazeutischen Zusammensetzung, und
- ein Kupfersalz mit einer Konzentration von 0,01 bis 0,015 mg Cu²⁺-lonen/ml der pharmazeutischen Zusammensetzung, **dadurch gekennzeichnet, dass** sie ferner ein Zinksalz mit einer Konzentration von 0,01 bis 0,015 mg Zn²⁺-Ionen/ml der pharmazeutischen Zusammensetzung enthält.

2. Pharmazeutische Zusammensetzung nach Anspruch 1, wobei das Kupfersalz ein Kupfernitrat und bevorzugt ein Kupfer(II)-nitrat-Trihydrat ist.

3. Pharmazeutische Zusammensetzung nach Anspruch 1 oder Anspruch 2, die ferner Zinknitrat enthält.

4. Verfahren zur Herstellung einer pharmazeutischen Zusammensetzung zur Behandlung von Hautkrankheiten, enthaltend, in wässriger Lösung, eine therapeutische Menge an Salpetersäure mit 5 bis 15 mmol/ml der pharmazeutischen Zusammensetzung, enthaltend:
- Zugeben einer Menge an metallischem Zink mit einer Konzentration von 0,01 bis 0,015 mg Zn²⁺-Ionen/ml zu einer vorbestimmten Menge konzentrierter Salpetersäure,
- Rühren der vorbestimmten Menge an Salpetersäure, die das Zink enthält, bis zu dessen Auflösung,
- Freisetzen des gasförmigen Wasserstoffs mit einer Reduktion der Salpetersäure in salpetrige Säure und Bildung einer ersten Lösung
- Zugeben einer Menge an Kupfer, insbesondere in Form eines Kupfernitrats mit einer Konzentration von 0,01 bis 0,015 mg Cu²⁺-Ionen/ml,
- Zugeben mindestens einer anderen Carbonsäure, bevorzugt ausgewählt aus Essigsäure, Oxalsäure und Milchsäure und Reduzieren der mindestens einen Carbonsäure unter Bildung von Nitriten, und
- Verdünnen mit Wasser, wobei das Zugeben einer Menge an Kupfer
a) in der ersten Lösung, oder
b) in einer anderen vorbestimmten Menge an konzentrierter Salpetersäure, unter Bildung einer zweiten Lösung, gefolgt vom Mischen der ersten Lösung mit der zweiten Lösung, vor dem Verdünnen mit Wasser durchgeführt wird.

5. Medizinische Vorrichtung, die einen Applikator und eine Ampulle enthält, wobei die Ampulle eine pharmazeutische Zusammensetzung nach einem der Ansprüche 1 bis 3 enthält.

6. Medizinische Vorrichtung nach Anspruch 5, wobei der Applikator eine Glaspipette ist, die angeordnet ist, um die pharmazeutische Zusammensetzung aus der Ampulle zu entnehmen, um sie auf die Hautläsion aufzutragen.

7. Medizinische Vorrichtung nach Anspruch 5, wobei der Applikator einen Behälter, der aus der Ampulle besteht, und ein Auftragsende enthält, das aus einer Kapillare besteht, die ein Ende aufweist, das aus einem abgeschrägten Ende, einem abgestumpften Teil, einem Pinsel, einem Aufsatz zum Auftragen, einem Auftragsschwamm, einer Hohlnadel ausgewählt ist.

8. Medizinische Vorrichtung nach Anspruch 5, wobei der Applikator einen Behälter, der angeordnet ist, um den Inhalt der Ampulle und ein Auftragsende aufzunehmen, enthält, das aus einer Kapillare gebildet wird, die ein Ende aufweist, das aus einem abgeschrägten Ende, einem abgestumpften Teil, einem Pinsel, einem Aufsatz zum Auftragen, einer Hohlnadel ausgewählt ist.

9. Pharmazeutische Zusammensetzung nach einem der Ansprüche 1 bis 3 zur Behandlung von Hautkrankheiten oder Hautläsionen, wie z. B. Dermatosen, Keratosen, Warzen, Condylomata, Ekzem, Hyperkeratosen, Akne, Psoriasis, Läsionen, die von Pilzinfektionen, bakteriellen oder viralen Infektionen stammen.
